# EUROPEAN PATENT SPECIFICATION

(11) **EP 1 845 899 B1**
(45) Date of publication and mention of the grant of the patent: **29.06.2011**
(21) Application number: 04806542.9
(22) Date of filing: 29.12.2004
(51) Int. Cl.: A61F 2/16

(54) **METHOD OF PREPARING A PRELOADED IOL INJECTOR**
METHODE ZUR VORBEREITUNG EINES VORBELADENEN INTRAOKULARLINSENINJEKTORS
MÉTHODE DE PRÉPARATION D'INJECTEUR PRÉCHARGÉ DE LENTILLE INTRAOCULAIRE

(43) Date of publication of application: 24.10.2007
(73) Proprietor: BAUSCH & LOMB INCORPORATED, Rochester, NY 14604 (US)
(72) Inventor: PYNSON, Joël, F-31400 Toulouse (FR)
(74) Representative: Vossius & Partner
(86) International application number: PCT/IB2004/004383
(87) International publication number: WO 2006/070219

(56) References cited:
- WO-A-01/64147
- WO-A-99/33411
- WO-A-2005/030097
- DE-U1- 20 219 445
- US-A- 5 807 400

## Description

### Background of the Invention

The present invention relates to ophthalmic methods. More particularly, the present invention relates to a method of preparing an intraocular lens (hereafter referred to as "IOL") injector for use, wherein the IOL may be conveniently preloaded in a component of the injector device for assembly at the time of surgery and without requiring direct handling of the IOL.

IOLs are artificial lenses used to replace the natural crystalline lens of the eye when the natural lens has cataracts or is otherwise diseased. IOLs are also sometimes implanted into an eye to correct refractive errors of the eye in which case the natural lens may remain in the eye together with the implanted IOL. The IOL may be placed in either the posterior chamber or anterior chamber of the eye. IOLs come in a variety of configurations and materials. Some common IOL styles include the so-called open-looped haptics which include the three-piece type having an optic and two haptics attached to and extending from the optic; the one-piece type wherein the optic and haptics are integrally formed (e.g., by machining the optic and haptics together from a single block of material); and also the closed looped haptic IOLs. Yet a further style of IOL is called the plate haptic type wherein the haptics are configured as a flat plate extending from opposite sides of the optic. The IOL optic and haptics may be made from a variety of materials or combination of materials such as PMMA, silicone, hydrogels and silicone hydrogels, acrylic, etc.

Various instruments and methods for implanting the IOL in the eye are known. In one method, the surgeon simply uses surgical forceps having opposing blades which are used to grasp the IOL and insert it through the incision into the eye. While this method is still practiced today, more and more surgeons are using more sophisticated IOL inserter devices which offer advantages such as affording the surgeon more control when inserting the IOL into the eye. IOL inserter devices have recently been developed with reduced diameter insertion nozzles which allow for a much smaller incision to be made in the eye than is possible using forceps alone. Smaller incision sizes (e.g., less than about 3mm) are preferred over larger incisions (e.g., about 3.2 to 5+mm) since smaller incisions have been attributed to reduced post-surgical healing time and complications such as induced astigmatism.

Since IOLs are very small and delicate articles of manufacture, great care must be taken in their handling. In order for the IOL to fit through the smaller incisions, they need to be made from a flexible material such as silicone or acrylic, for example, and folded or compressed prior to entering the eye wherein they will assume their original unfolded/ uncompressed shape. The IOL inserter device must therefore be designed in such a way as to permit the non-destructive loading of the IOL into the inserter, as well as easy passage of the IOL through the device and into the eye. Should the IOL be damaged during loading thereof into the inserter or during delivery into the eye, the surgeon will most likely need to extract the damaged IOL from the eye and replace it with a new IOL, a highly undesirable surgical outcome.

Thus, as explained above, the IOL inserter device must be designed to permit non-destructive loading thereof into an inserter as well as easy passage of the IOL therethrough. It is equally important that the IOL be expelled from the nozzle of the IOL inserter device and into the eye in a predictable orientation and manner. Should the IOL be expelled from the nozzle too quickly or in the wrong orientation, the surgeon must further manipulate the IOL in the eye which could result in trauma to the surrounding tissues of the eye. It is therefore highly desirable to have an inserter device which allows for precise loading of the IOL into the inserter device and which will pass and expel the IOL from the inserter device nozzle and into the eye in a controlled, predictable and repeatable manner.

To ensure controlled expression of the IOL through the nozzle of the IOL inserter device, the IOL must first be loaded into the IOL inserter device. The loading of the IOL into the inserter device is therefore a precise and very important step in the process. Incorrect loading of an IOL into the inserter device is oftentimes cited as the reason for a failed IOL delivery sequence. Many IOL injector devices on the market today require the IOL to be directly handled for loading into the injector at the time of surgery by the attending nurse and/or surgeon. Due to the delicate nature of the IOL, there is a risk that the nurse and/or surgeon will inadvertently damage the IOL and/or incorrectly loading the IOL into the injector device resulting in a failed implantation. Direct handling and/or loading of the IOL into the injector by the nurse and/or surgeon is therefore undesirable.

In a typical IOL inserter device, the IOL inserter utilizes a plunger having a nozzle which engages the IOL (which has been previously loaded and compressed into the inserter lumen) to pass the IOL through the inserter lumen. The IOL thus interfaces with the plunger tip as well as the lumen of the inserter device. The lumen typically is dimensioned with a narrowing toward the open tip of the nozzle in order to further compress the IOL as it is advanced through the lumen. The nozzle tip of the lumen is sized for insertion through the surgical incision which, as stated above, is presently preferred in the sub 3mm range. Thus, an inserter lumen will typically be dimensioned larger at the loading area of the IOL and gradually decrease in diameter to the nozzle open tip of the lumen where the IOL is expressed into the eye. It will be appreciated that the compressed diameter of the IOL at the lumen nozzle open tip is the same as the inner diameter of the lumen nozzle open tip, preferably sub 3mm as stated above. Each of these component interfaces are dynamic in the sense that the forces acting between the interfacing components (i.e., the IOL, the plunger tip and the inserter lumen) will vary as the IOL is pushed through the lumen.

There remains a need for a method which removes the need for direct handling of the IOL by the nurse and/or surgeon and which generally simplifies operation of the IOL injector device and IOL delivery process. The document WO99/33411 discloses a method of operating an IOL injector comprising a body and a nozzle.

### Summary of the Invention

The invention according to the claims can be performed with an injector for injecting an IOL into an eye wherein the injector includes an injector nozzle which is pivotally attached to an injector body and pivotably movable with respect thereto between an IOL loading position and an IOL injection position. When in the injection position, the distal tip end of the injector nozzle is in position for inserting the IOL into an eye. The injector nozzle may be rotated with respect to the injector body such that when in the IOL loading position, the proximal end of the injector nozzle is accessible for loading of the IOL therein.

The IOL is preloaded into a separate shuttle component of the injector device and placed in a vial of storage solution at manufacture. At the time of use, the surgeon or nurse first ensures the injector nozzle is in its IOL loading position relative to the injector body to which it is pivotally attached. The surgeon/nurse opens the vial and places the proximal end of the injector nozzle into the vial to attach the shuttle component to the injector nozzle and then withdraws the nozzle and shuttle with IOL from the vial. In an alternate embodiment, the shuttle is removed from the vial and then attached to the injector nozzle. The surgeon/nurse then pivots the injector nozzle on the injector body to the insertion position whereupon the injector device is ready for injecting the IOL into an eye. In this way, there is no need for the surgeon/nurse to directly handle the IOL since it is already preloaded in the shuttle component when received from the manufacturer.

### Brief Description of the Drawing

Figure 1A is a perspective view of a first embodiment to be used for the invention showing the shuttle and IOL in the vial and the injector body spaced therefrom;

Figure 1B is the view of Figure 1A showing the empty vial and the shuttle and IOL connected to the injector nozzle in the storage position;

Figure 1C is the view of Figure 1B showing the injector nozzle being moved to the injection position;

Figure 1D is the view of Fig. 1C showing the injector nozzle moved to the injection position;

Figure 2A is a perspective view of a second embodiment to be used for the invention showing the shuttle and IOL in the vial and the injector body spaced therefrom;

Figure 2B is the view of Figure 2A showing the empty vial and the shuttle and IOL connected to the injector nozzle in the storage position;

Figure 2C is the view of Figure 2B showing the injector nozzle being moved to the injection position;

Figure 2D is the view of Fig. 2C showing the injector nozzle moved to the injection position;

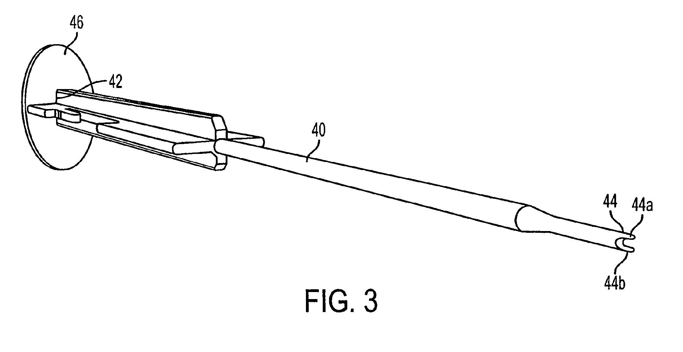

Figure 3 is a perspective view of an embodiment of a plunger of the injection device;

Figure 4A is a perspective view of an embodiment of the shuttle of the injection device showing an embodiment of IOL loaded in the open shuttle; and

Figure 4B is a perspective view of the shuttle in the closed position and oriented 180° relative to the view of Fig. 4A.

### Detailed Description

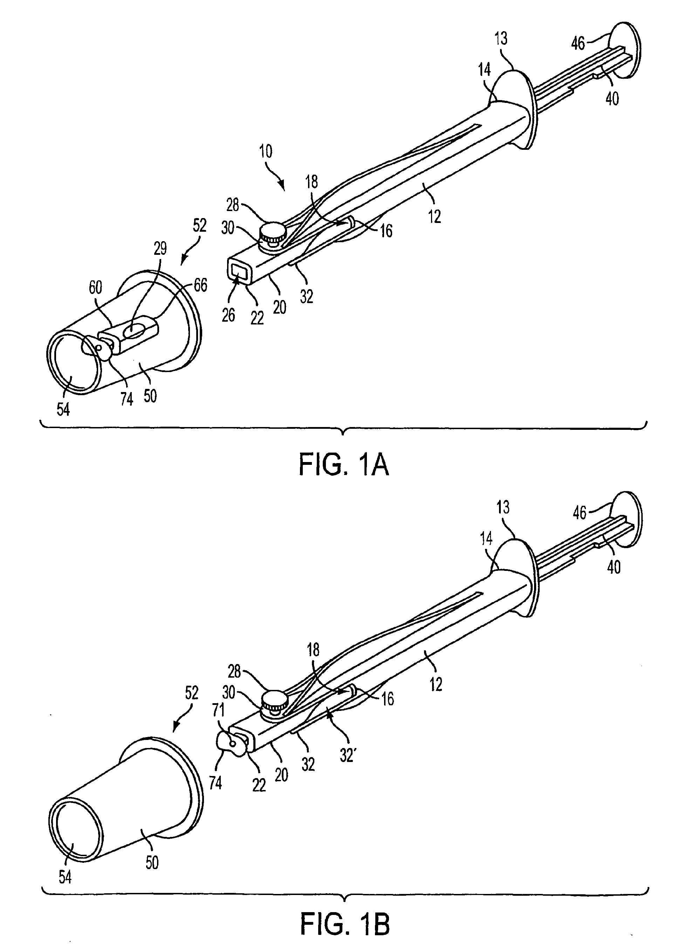

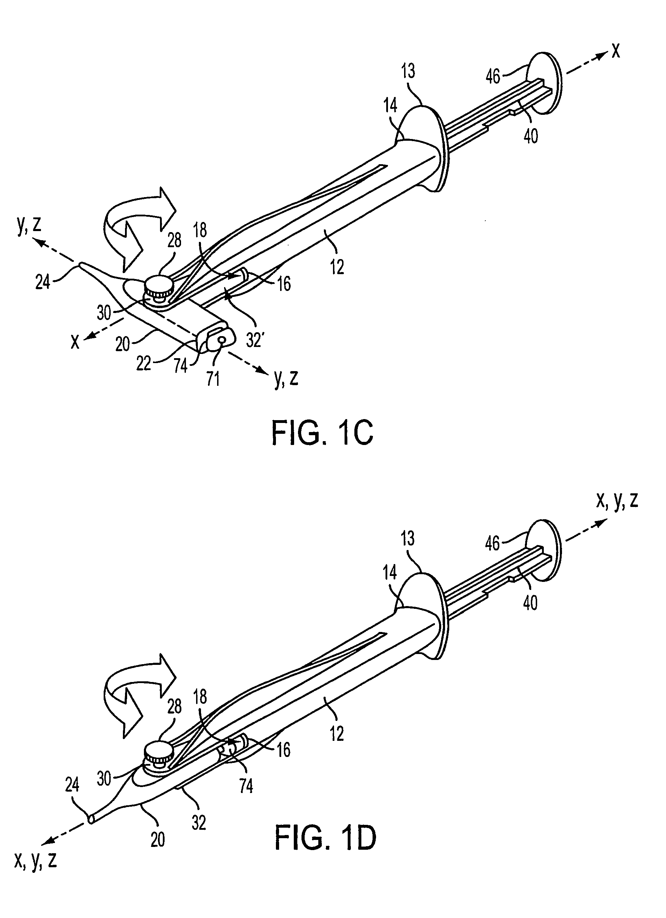

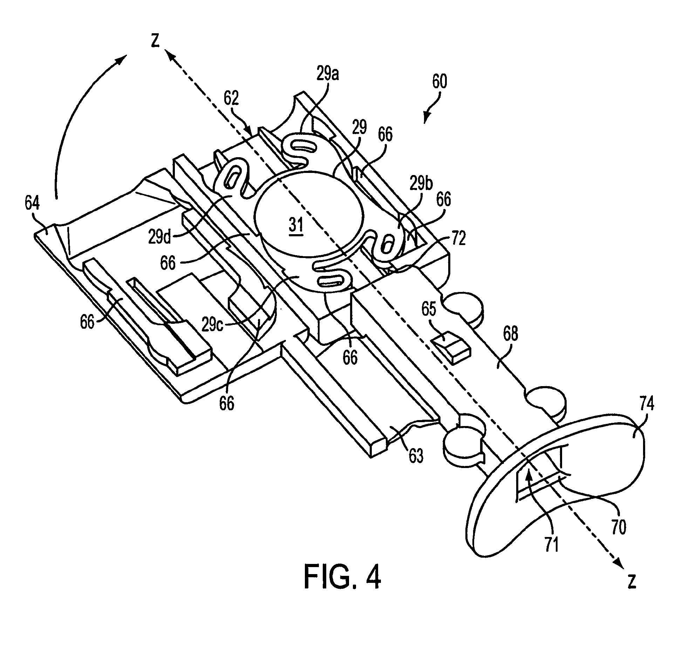

In a first aspect, the invention uses an injector device 10 for injecting an IOL 29 (seen best in Fig. 4) into an eye, injector 10 including an injector body 12 having respective proximal and distal ends 14, 16 and a longitudinal opening 18 extending along an axis X-X therebetween. An injector nozzle 20 having respective proximal and distal ends 22, 24 and a longitudinal opening 26 extending along an axis Y-Y therebetween is pivotally connected to body 12 near the body distal end 16. The injector nozzle 20, body 12 and pivotal connection therebetween may take a variety of configurations and is not limited to the embodiment shown in the drawings. In the embodiment of Figs. 1A-1D, injector body extensions 30, 32 extend in spaced, parallel relation from the distal end 16 of body 12 defining spaced slots 30', 32' (only 32' is seen in Figs. 1A-1D). Injector nozzle 20 is positioned between extensions 30, 32 and a pair of pivot pins 28 (only one pin 28 is seen in Figs. 1A-1D) extend through extensions 30, 32 and into the wall of injector nozzle 20, respectively. It is, of course, understood that the pivot pins 28 do not extend into the nozzle longitudinal opening 26 to an extent that would interfere with the IOL passage therethrough. Nozzle 20 is thus pivotally movable about pins 28 between an IOL loading position as seen in Figs. 1A and 1B, and an IOL injection position as seen in Fig. 1D. The loading and injection positions are 180° offset from each other although this may vary somewhat depending on the particular injector design employed. When in the IOL loading position, the IOL 29 may be loaded into the injector nozzle 20. When in the injection position, the IOL 29 may be expressed from the distal tip end 24 of nozzle 20. To advance the IOL 29 through and out of the nozzle 20, a plunger 40 is provided in longitudinal opening 18 for telescoping movement therein. IOL advancement within and out of nozzle 20 will be described in more detail below.

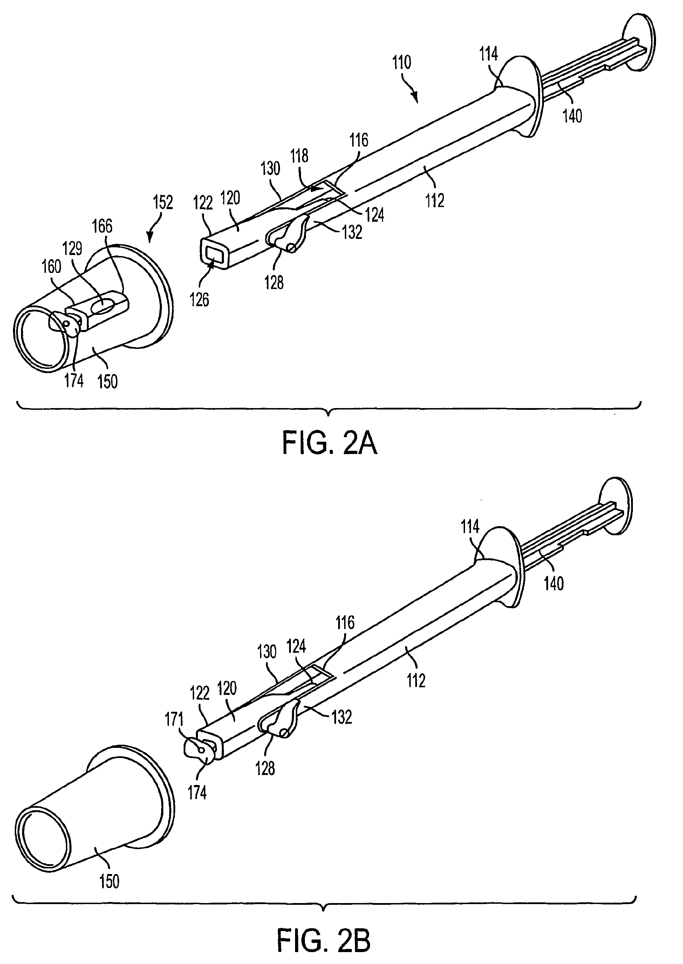

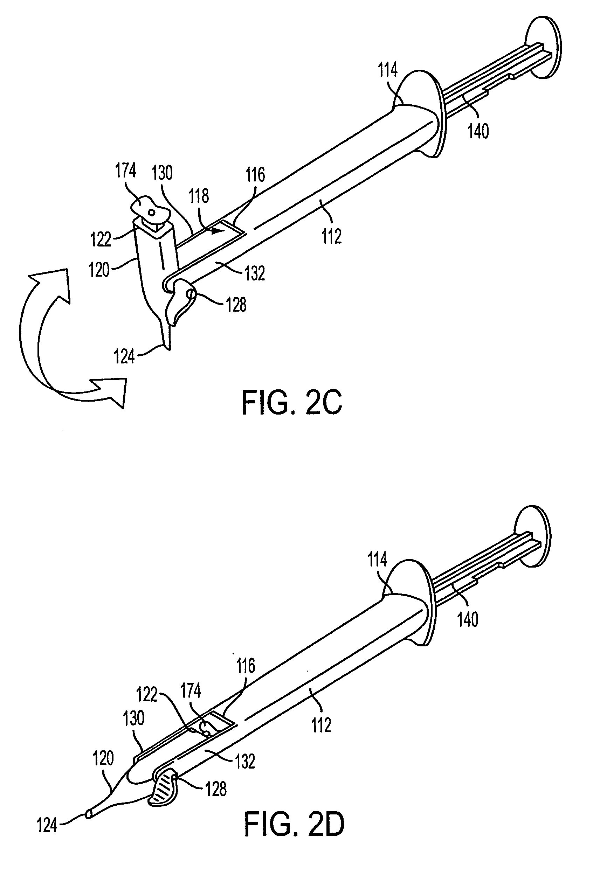

Referring to Figs. 2A-2D, another embodiment of an injector device is seen where the main difference in this embodiment is that the pivot connection is 90° offset from that of the embodiment of Figs. 1A-1D. Like parts in Figs. 2A-2D are therefore indicated with like numerals increased by a factor of 100.

Referring again to Figs. 1A-1D, a container such as a vial 50 is provided in which an IOL such as IOL 29 may be stored and shipped. The IOL may be made of a hydrophilic material such as acrylic which requires storage in solution. It is understood, however, that IOLs of any material (requiring wet or dry storage) and configuration are within the scope of the invention. In the present embodiment, a quantity of storage solution (not shown) is therefore also deposited in vial 50 to maintain IOL 29 hydrated prior to use. The vial is sealed and sterilized according to known techniques.

The IOL 29 must be presented in the vial 50 in a stable orientation to allow the transfer thereof from the vial 29 to the injector nozzle 20 without requiring other IOL handling tools such as a forceps. As seen best in Fig. 4, IOL 29 is preferably held in a shuttle component 60 which is inserted, together with IOL 29, into the vial 50 with storage solution. Shuttle component 60 is configured to hold and store IOL 29 in a non-destructive, unstressed manner and may vary from the configuration shown to accommodate the particular IOL configuration to be held thereby. In the embodiment of Fig. 4, IOL 29 is configured with an optic 31 and four closed loop haptics 29a-d extending from optic 31. Shuttle component 60 includes an IOL loading platform 62 having a cover 64 which may be moved to the closed position about living hinge 63 to encase the IOL 29 between cover 64 and platform 62. This style of shuttle component is also disclosed in our copending PCT application WO2005/030097 serial number PCT/IB03/04686 filed on September 26, 2003) published on April 4, 2005.

Cooperatively configured IOL locating features 66 in the form of raised, contoured wall areas may be provided on one or both of the platform 62 and cover 64 to correctly position and stabilize IOL 29 within the shuttle 60. A neck portion 68 extends from platform 62 and includes a longitudinal opening 71 extending from the neck proximal end 70 to the neck distal end 72 which opens to the space between platform 62 and cover 64 when closed. The IOL 29 is loaded onto platform 62 with the optic 31 thereof aligned along the longitudinal axis Z-Z of shuttle 60. The proximal end 70 of the shuttle neck 68 includes a collar 74 which is positioned adjacent the vial bottom 54 at the point of manufacture.

At the time of surgery, the surgeon/nurse removes the seal from the vial 50 to expose the open top 52 thereof. With the injector nozzle 20 in the IOL loading position seen in Fig. 1A, the proximal end 22 of the nozzle 20 is brought into engagement with shuttle distal end 66 and the platform 62 and closed cover 64 are inserted into nozzle proximal opening 26. When shuttle 60 is attached to nozzle 20, the longitudinal axis Z-Z of shuttle 60 is aligned along the longitudinal axis Y-Y of the nozzle 20. In a preferred embodiment, the shuttle component 60 is mechanically locked to the nozzle 20 by any desired means, for example, a boss 65 provided on neck 68 which automatically engages a recess (not shown) on the inside surface of the nozzle 20. The nozzle 20 and shuttle 60 (with IOL 29 therein) may then be together withdrawn from the now empty vial 50 as seen in Fig. 1B. In this embodiment, the collar 74 of the shuttle neck 68 extends from nozzle proximal end 22 although the shuttle may be completely encased within the nozzle component if desired. The injector nozzle 20 is now ready to be moved to the injection position which is accomplished by pivoting nozzle 20 with respect to body 12 about pivot pins 28. If desired, manual pivoting of the nozzle is facilitated through a wheel 28a or lever 128a or the like. Suitable cooperative locking means (not shown) may be provided on the nozzle 20 and body 12 to secure the nozzle in the injection position. Such locking means may engage automatically when the nozzle is pivoted to the injection position.

In an alternate embodiment, the shuttle 60 may be removed from vial 50 prior to being attached to nozzle 20 (not shown). The shuttle 60 may be handled manually without contacting the IOL 29. Alternatively, the shuttle 60 may be releasably attached to the seal/cover of the vial which is used for handling the shuttle. Once the shuttle is attached to the nozzle, the seal/cover is released from the shuttle end 74. Other shuttle handling instruments are of course possible (e.g., forceps).

Fig. 1C shows the nozzle 20 being moved from the IOL loading position of Fig. 1B to the IOL injection position of Fig. 1D. Clearance for movement of nozzle 20 is provided by the slots 30', 32' defined between the injector body extensions 30, 32. When in the injection position, the axis Y-Y of longitudinal opening 26 of nozzle 20 and axis Z-Z of shuttle opening 71 are aligned with the longitudinal opening 18 of the body 12 (see Fig. 1D). Upon advancing plunger 40, the plunger tip 44 enters shuttle neck 68 through longitudinal opening 71 at neck proximal end 70. In this regard, in a preferred embodiment, plunger 40 includes a proximal end 42 and distal tip 44 configured to non-destructively engage and push IOL 29 through and out of the injector nozzle 20. Plunger proximal end 42 may be provided with a thumb press 46, and injector body 12 may be provided with a finger flange 13 such that the plunger may be advanced in body 12 in the manner of a syringe. Upon continued advancement of the plunger 40, the plunger tip 44 enters the area between the platform 62 and cover 64 and engages the edge of the IOL optic 31 between plunger tip spaced ends 44a and 44b. Further advancement of the plunger 40 pushes the IOL 29 out of the shuttle 60, through nozzle 20, and ultimately exiting at nozzle distal tip end 24. The nozzle opening 26 narrows toward end 24 to compress the IOL 29 as it is advanced therethrough so that it may pass through the narrow nozzle 24 and into the eye whereupon IOL 29 regains its original shape.

The invention thus provides a method of preparing an injector device in which direct handling of the lens is not required, the loading of the lens into the injector is reliable, and no contact is required between the operator's hands and the nozzle tip.

## Claims

1. A method of preparing an IOL injector for use, said method comprising the steps of:
a) providing an injector device (10, 110) having an injection body (12, 112);
b) providing a container (50) having a removable cover;
c) providing a shuttle (60) removably attached to said cover, the shuttle (60) removably holding an IOL (29), said shuttle (60) and said IOL (29) being removably stored within said container (50) of storage solution;
d) removing said shuttle (60) from said container (50) using said cover; and
e) attaching said shuttle (60) to said injector device using said over.

2. The method of claim 1, wherein said shuttle (60) is attached to the injection body (12, 112).

3. The method of claim 1, wherein said shuttle (60) is attached to a nozzle (20, 120) of said injector device.

4. The method of any of claims 1 to 3, wherein said container holds a quantity of storage solution therein.

## Patentansprüche

1. Verfahren zur Vorbereitung eines Intraokularlinseninjektors zur Verwendung, wobei das Verfahren die folgenden Schritte aufweist:
a) Bereitstellen einer Injektorvorrichtung (10, 110) mit einem Injektorkörper (12, 112);
b) Bereitstellen eines Behälters (50) mit einem lösbaren Deckel;
c) Bereitstellen eines Shuttles (60), das lösbar am Deckel befestigt ist, wobei das Shuttle (60) lösbar eine Intraokularlinse (29) hält und wobei das Shuttle (60) und die Intraokularlinse (29) lösbar im Behälter (50) für Aufbewahrungslösung gelagert sind;
d) Lösen des Shuttles (60) vom Behälter (50) mittels des Deckels; und
e) Befestigen des Shuttles (60) an der Injektorvorrichtung mittels des Deckels.

2. Verfahren nach Anspruch 1, wobei das Shuttle (60) am Injektorkörper (12, 112) befestigt ist.

3. Verfahren nach Anspruch 1, wobei das Shuttle (60) an einer Düse (20, 120) der Injektorvorrichtung befestigt ist.

4. Verfahren nach einem der Ansprüche 1 bis 3, wobei im Behälter eine Menge an Aufbewahrungslösung enthalten ist.

## Revendications

1. Procédé pour préparer un injecteur de IOL destiné à être utilisé, ledit procédé comprenant les étapes consistant à :
a) prévoir un dispositif formant injecteur (10, 110) ayant un corps d'injection (12, 112) ;
b) prévoir un récipient (50) ayant un couvercle amovible ;
c) prévoir une navette (60) fixée de manière amovible sur ledit couvercle, la navette (60) maintenant de manière amovible une IOL (29), ladite navette (60) et ladite IOL (29) étant stockées de manière amovible à l'intérieur dudit récipient (50) de solution de stockage ;
d) retirer ladite navette (60) dudit récipient (50) à l'aide dudit couvercle ; et
e) fixer ladite navette (60) sur ledit dispositif formant injecteur à l'aide dudit couvercle.

2. Procédé selon la revendication 1, dans lequel ladite navette (60) est fixée sur le corps d'injection (12, 112).

3. Procédé selon la revendication 1, dans lequel ladite navette (60) est fixée à une buse (20, 120) dudit dispositif formant injecteur.

4. Procédé selon l'une quelconque des revendications 1 à 3, dans lequel ledit récipient contient une quantité de solution de stockage à l'intérieur de ce dernier.
